# EUROPEAN PATENT APPLICATION

(11) **EP 2 783 695 A1**
(43) Date of publication of application: **01.10.2014**
(21) Application number: 13161748.2
(22) Date of filing: 28.03.2013
(51) Int. Cl.: A61K 38/04, A61K 9/00, A61K 31/185, A61K 31/205, A61K 31/355, A61K 31/728, A61P 27/02

(54) **Physiological supplement or medicament for ophthalmic use containing L-carnitine or alkanoyl L-carnitines in combination with eledoisin**

(71) Applicant: SIGMA-TAU Industrie Farmaceutiche Riunite S.p.A., 00144 Roma (IT)
(72) Inventor: Pescosolido, Nicola, 00181 Rome (IT)
(74) Representative: Tagliafico, Giulia

(57) **Abstract**

Use of L-carnitine and/or of one or more alkanoyl L-carnitines or one of their pharmaceutically acceptable salts in combination with eledoisin, for the preparation of an opthalmic physiological supplement or medicament in the form of eye-drops, for the treatment of corneal diseases.

## Description

### FIELD OF THE INVENTION

The present invention relates to the use of L-carnitine and/or one or more alkanoyl L-carnitines, or a salt thereof, in combination with eledoisin, for the preparation of a physiological supplement or medicament in the form of eye-drops useful for the treatment of corneal diseases.

### BACKGROUND OF THE INVENTION

The cornea is the transparent, dome-shaped window covering the front of the eye. It is a powerful refracting surface, providing 2/3 of the eye's focusing power. Like the crystal on a watch, it gives us a clear window to look through.

The cornea is extremely sensitive - there are more nerve endings in the cornea than anywhere else in the body.

The adult cornea is only about ½ millimeter thick and is arranged in three main regions, or layers:
- Epithelium: it functions primarily to block the passage of foreign material such as dust or water into the eye, and other layers of the cornea, and provide a smooth surface that absorbs oxygen and other needed cell nutrients that are contained in tears. This layer, which is about five cells deep, is filled with thousands of tiny nerve endings that make the cornea extremely sensitive to pain when rubbed or scratched.
- Stroma: is located behind the epithelium, the stroma comprises about 90 percent of the cornea. It consists primarily of water and layered protein fibers that give the cornea its strength, elasticity, and form; and cells that nourish it. The unique shape, arrangement, and spacing of the protein fibers are essential in producing the cornea's light-conducting transparency.
- Endothelium: this single layer of cells is located between the stroma and the aqueous humor. Because the stroma tends to absorb water, the endothelium's primary task is to pump excess water out of the stroma. Without this pumping action, the stroma would swell with water, become hazy, and ultimately opaque.

Many diseases can damage this delicate structures.

The main causes of impairment of the epithelial structure of the cornea are dry eye syndrome; corneal abrasions and injuries due to, for example, the application of contact lenses; and refractive laser surgery.

Other diseases of the cornea are associated with impairment of the normal transparency of the corneal surface, caused, for example, by damage in the aftermath of keratitis, particularly bacterial, viral or fungal keratitis; by damage resulting from trauma and refractive laser surgery; as well as degenerative or hereditary diseases such as chronic and acute keratoconus.

The tear film, which coats the corneal epithelium and is essential for the homeostasis of the eye surface, performs an important optical function, acting as a lubricant between the eyelids and the eyeball and as a vehicle for oxygen, guaranteeing the metabolism of the cells of the corneal epithelium; it also performs a flushing function, ensuring the removal of external agents. The normal tear osmolality in adult (human) is about 300 ± 10.0 mOsm/kg and is not significantly affected by age (Optom. Vis. Sci. 1995, vol 72, n° 10, 713-717).

The tear film is also important for its function as a carrier for growth factors, neuropeptides, and neuromodulators that regulate the activation, proliferation and differentiation of corneal and conjunctival epithelial cells. It also transports immunoglobulins (IgA, IgAs, IgG, IgE, IgM), complement factors (C3, C4, C5), metalloproteases (MMP-2, 4, 9), enzymes (lysozyme, lactoferrin) and immune system cells (lymphocyte), thus performing a fundamental defensive function against infections.

Dry eye syndrome is characterised by a quantitative (hypolacrimation) and/or qualitative (dyslacrimation) impairment of the tear film of multifactorial origin which may or may not cause clinically significant damage to the eye surface. The prevalence of dry eye syndrome ranges from 10 to 40% in the adult population and there is a highly significant correlation with age.

In the United States, the prevalence of mild-to-moderate dry eye syndrome is up to approximately 10 million people (Am. J. Ophthalmol:, 1997;124:723-728*;* Arch Ophthalmol., 2000; 118: 1264-1268).

Various studies conducted in order to understand the mechanisms activated in this disease have shown that the tears of subjects affected by dry eye syndrome present: an increased evaporation rate, increased surface tension, reduced vitamin A concentration, increased osmolality, reduced concentration of a number of proteins (lysozyme, lactoferrin), insufficient mucus production or qualitative changes in mucus production, with consequent inadequate reconstruction of the mucus layer, reductions in a number of growth factors (EGF, TGF-a, aFGF-bFGF, LG-F, HGF) (Contactologia, 1982; 4: 34-37), changes in concentration of inorganic elements, reduced androgens and dysregulation of T lymphocyte activity (Cornea, 2005; 24: 1-7). Among the mechanisms activated in this disease the use of contact lenses has to be mentioned.

The clinical signs regarded as being most closely related to this pathological condition are reduced break-up time (BUT test) and Schirmer test results (Pescosolido N.: Le alterazioni del film lacrimale. In Stendler P.: "il sistema lacrimale", Fabiano editore, Canelli (AT), 2000; pag. 237-330*;* hereinafter this reference will be referred to as *Pescosolido 2000*).

The BUT test has to do with the mucin content of the tear film and, in the dry eye, yields only values below 5 seconds. The Schirmer test, on the other hand, has to do with the water content of the tear film and, in the dry eye, yields values below 5 millimeters in 5 minutes.

The patient presents the following symptoms: foreign body sensation, burning, difficulty blinking, bruit on opening the eyelids, itching, eye fatigue, photophobia, blurred vision, and mucus extravasation at the inner canthi.

The treatment of this syndrome is based on the use of the following tear substitutes whose task is the regular moisturizing of the cornea, but which do not exert any action on the basic causes of the disease and are endowed only with very short-lasting efficacy; inserts (plugs) in the lacrimal canaliculus; immunoregulators such as topical cyclosporin; topical steroids; anti-inflammatory agents (rumexilone and loteprednol); autologous serum (cytokine inhibitors); topical or systemic androgens; mucus (HETE eicosanoid) and aqueous (P2Y2 agonists) secretogenic substances; acquaporins and agents such as antibiotics and detergents for the treatment of a disease often concomitant, blepharitis (Cornea, 2005; 24: 1-7).

Also used is treatment with iodide iontophoresis owing to its scavenger activity as a reducing agent and electron donor (Adv. Clin. Path., 2000; 4: 11-17*;* Br. J. Ophthalmol., 2005; 89: 40-44).

Even these latter treatments, despite exerting an action which may be regarded as more relevant to treating the causes of the disease, have failed to yield the anticipated results.

The normal transparency of the corneal surface can be impaired by the aftermath of numerous diseases acquired, degenerative or congenital that damage the delicate structure of the various constituent components. The acquired disease conditions most commonly implicated are post-keratitis damage, particularly after herpetic keratitis, and damage occurring in the aftermath of trauma and laser refractive surgery. The minimum common denominator is the formation of corneal opacities (leucomas) that functionally jeopardise vision. The events involved in wound healing that occur in the corneal tissue after infection, caustic damages, injury and refractive ablative surgery are have a profound effect on the final morphological and refractive outcomes of the restitutio ad integrum process.

The acute epithelial and stromal corneal lesions occurring immediately after injury and laser ablation are probably involved in the regulation of the subsequent corneal tissue repair events, and, among the latter, keratocyte apoptosis probably plays a major role (Cornea, 2000;19:S7-12). This event is responsible for the corneal repair process since keratocyte apoptosis is the prime mover of the reproliferative stimulus. The stroma keratocytes underlying the initial acellular stroma therefore represent the cell source that mediates the subsequent healing of the surface stroma beneath the epithelium. As a result of the cellular repopulation, the activated keratocytes undergo myofibroblastic transformation (Invest. Ophthalmol. Vis. Sci., 1998;39:487-501), thus proving responsible for the production of collagen fibres and of basic substances involved in the restituito ad integrum process.

A recent scar is very similar to fetal tissue, it is rich in fetal and type III collagen with thick fibrils, wide interfibrillary spaces and a hydrophilic stroma. This explains the characteristic opacity. It also contains fibrinogen, fibronectin, laminin and weakly sulphurized keratan sulphate. The keratan sulphate/condroitin sulphate is reduced in the scar stroma.

The myofibroblasts disappear in a few weeks to a few months. Gradually the scar changes and becomes less opaque. The resistance of the stromal scar tissue is 20% compared with normal tissue after three weeks, 60% after 1 year and 70% after 3-4 years. Corneal reinnervation is very slow. The cytokines play an important role in these events. IL-1a, produced by the corneal epithelium, stimulates metalloprotease and collagenase synthesis through the keratocytes and the myofibroblasts. The TGF-β reduces the production of collagenase and is involved in the formation of haze. IL-6 reduces the synthesis of metalloprotease thus increasing collagen synthesis.

This process, however, is not self-controlled and, in many cases, abnormal, excessive healing occurs followed by a greater production of collagen type III, an increase of jaluronic acid, and an increase in lamellar disorganisation (Arch. Ophthalmol., 1990; 108: 665-675*;* Exp. Eye Res., 2004; 78 : 553-560*;* Ophthalmology, 2000; 107 : 1235-1245) or an accumulation of keratocytes and myofibroblasts as shown by confocal microscopy (Progr. Retin. Eye Res., 1999; 18:311-356*;* J. Cataract Refract. Surg., 2000; 26 : 432-447*;* Exp. Eye Res., 2004; 78 : 553-560).

These abnormalities are involved in the pathogenesis of the most feared complication of stromal regeneration after photorefractive keratectomy (PRK), namely, haze, with consequent impairment of the functional outcome. Haze is classified according to Heitzmann in 5 degrees on the basis of the visual impairment due to the reduced corneal transparency(Ophthalmology, 1998; 35 : 1411-1421); or according to the Fantes scale (Arch. Ophthalmol., 1990; 108 : 665-675) which distinguish 6 degrees of haze (0; 0.5; 1+; 2+; 3+; and 4+). Though the incidence of haze has been substantially reduced over recent years, as a result of the technological advances in the field of excimer lasers, it is still a fairly frequent complication even today and, in rare cases, would appear hard to reverse, even after months of cortisone therapy (0,5% of the eye with myopia not exceeding 6 diopters and 3-17% in the eye with myopia exceeding 10 diopters (Ophthalmol. Clin. North. Am., 2001, 14 : 359-376). In cases of persistent haze (more than 15-18 months) which fails to respond to medical cortisonic therapy [an event that can occur with late-onset haze (4-12 months after the operation *(*Ophthalmology, 1997; 104 : 369-374*;* Cornea, 2004, 23 : 350-355*),* as the only feasible procedure is phototherapeutic keratectomy (PTK) with an excimer laser, a procedure used for the laser-assisted surgical removal of superficial stromal opacities combined with 0.02% mitomycin (J. Refract. Surg., 2003;19:40-43; J. Refract Surg., 2003,19:449-454). The use of the activator of plasminogen and of TGF-α1 is still undergoing animal studies (J. Refract. Surg., 1997;13:356-361; Invest. Ophthalmol. Vis. Sci., 2004;45:1329-1333), while INFβ-2b, tested on man, has produced poor results (J. Refract. Surg., 1996;22:891-900).

Indeed, while low degree haze is generally asymptomatic, or only causes a slight reduction in sensitivity to contrast, serious forms of haze can interfere with refraction and be accompanied by myopic regression due to exuberant scarring (J. Refract. Surg., 1995;11.341-347; Ophthalmology, 2000;107:1235-1245).

The corneal dystrophies (Bietti, 1971; Oftalmologia geriatrica) are rare disorders. They are defined as primitive corneal disorders not associated with trauma, earlier inflammation or systemic diseases. They affect both eyes, are hereditary and, for the most part, have an autosomal dominant trait.

Corneal degeneration is far more common than dystrophies but the symptoms are generally less obvious. It is not hereditary, can be unilateral or bilateral and the course is gradual or relatively stable. In each case, it is, by definition, permanent, or does not resolve itself spontaneously.

Usually it involves more than one layer of the cornea. Distinctions can be drawn between primary or idiopathic and secondary forms. The first are often connected with ageing without being preceded by any specific pathological process, the second ones are always associated with eye diseases which precede them, whether acute, chronic, infectious or inflammatory in nature. Keratoconus is one of the most typical degenerative diseases.

Keratoconus is a non-inflammatory ectasia of the paracentral portion of the cornea which evolves over time and takes a progressively conical form, with tapering of the tip. This corneal deformation results in severe astigmatism, often irregular, corrected wherever possible with hard gas-permeable corneal lenses. Only rarely are epikeratoplasty, photorefractive keratectomy or the use of intracorneal rings (INTACTS) considered (Cornea, 1987;6:131-139; Ophthalmology, 1992;99:1187-1192; Surv. Ophthalmol., 1998;42:297-319;J. Cataract. Surg., 2000; 26 : 1117-1122; J. Refract.Surg., 2001;17:69-73) or also the use of hard gas-permeable lenses combined with INTACTS. All these techniques only correct the refractive defects but do not resolve the cause of the corneal ectasia and therefore do not stop the progress of keratoconus, which in its most severe form means a corneal transplant (Cornea, 1997;16:623-629; Cornea, 1997;16:414-419; Cornea, 1998;17:468-470; Cornea, 2002;21:152-155 ).

Keratoconus starts at puberty and in 20% of cases perforating keratoplasty has to be performed (Ophthalmology, 1994; 101 : 439-447).

Past studies have not produced relevant results leading to an understanding of the physiopathology of the disease but only more recently, with the development of molecular techniques, have advances been made in understanding this abnormality.

At present, however, we are not able to say that the presence of keratoconus is due to uniform anomalies which are specific to the extracellular matrix. There are areas where the elements of the basal membrane are absent indicating on-going proteolytic activity, and there are areas in which there are deposits of fibrotic substance which is also found in other pathologies. A cornea with keratoconus has low levels of inhibitory enzymes (TIMP) and greater activity of the enzymes which are capable of degrading the extracellular matrix. The TIMP play a predominant role in the thickness of the stroma and in the stability of Bowman's membrane, which is characteristically interrupted in keratoconus. Furthermore the decrease in one of these inhibitors, TIMP-1, could play an important role in apoptosis or in the anomalous behaviour of the cells found in keratoconus.

Apoptosis is the programmed death of the cells. This death is necessary for reconstructing damaged cells and for the normal turn-over of many tissues.

In keratoconus, apoptosis is more frequently found in the stroma (Invest. Ophthalmol. Vis. Sci., 1998; 39 : 220-226) than in other corneal layers (Cornea, 2002; 21 : 206-209).

This observation is important because a cornea with keratoconus is subject to chronic irritation caused by hard gas-permeable contact lenses, to greater friction and to moderate or severe atrophy. Wilson suggests that mechanical trauma on the epithelium could cause apoptosis in the stromal cells of keratoconus (Exp. Eye Res., 1999; 69 : 225-266;Cornea, 2000; 19 : S7-S12). Furthermore, an increase in the levels of leukocytes commonly correlated to the LAR protein antigen present in keratoconus but not in normal corneas can be seen (Exp. Eye Res., 1999;68:283-293).

LAR is a transmembrane phosphotyrosine transferase capable of stimulating apoptosis (Proc. Nati. Acad. Sci. USA, 1994;91:10868-10872; J. Neurobiol., 2000;42:477-486). A third triggering mechanism of apoptosis is that it is inhibited by TIMP-1 (J. Clin. Invest., 1998;102:2002-2010; Blood, 1998;92:1342-1349) which in keratoconus proves to be reduced, as has been reported previously.

In practice, given these statements, the phenomenon of apoptosis could play an important role in the pathogenesis of keratoconus.

Eledoisin, IUPAC Name: 3-[[6-amino-2-[[3-hydroxy-2-[[1-(5-oxopyrrolidine-2-carbonyl)pyrrolidine-2-carbonyl]amino]propanoyl]amino] hexanoyl]amino]-4-[[1-[[1-[[1-[[2-[[1-[(1-amino-4-methylsulfanyl-1-oxobutan-2-yl)amino]-4-methyl-1-oxopentan-2-yl]amino]-2-oxoethyl]amino]-3-methyl-1-oxopentan-2-yl]amino]-1-oxo-3-phenylpropan-2-yl]amino]-1-oxopropan-2-yl]amino]-4-oxobutanoic acid; CAS Number: 69-25-0; is an undecapeptide of mollusk origin, belonging to the tachykinin family of neuropeptides, was first isolated from the posterior salivary glands of two mollusk species *Eledone muschata* and *Eledone aldovandi,* which belong to the octopod order of Cephalopoda. Other tachykinins from non-mammalian sources include kassinin and physalaemin. The mammalian tachykinins substance P, NKA, and NKB have similar effects as tachykinins of non-mammals and have been more widely studied and characterized. These peptides exhibit a wide and complex spectrum of pharmacological and physiological activities such as vasodilation, hypertension, and stimulation of extravascular smooth muscle.

Sodium hyaluronate is a very well known compound used to protect corneal epithelial cells, especially in patients with dry eye syndrome or with Sjögren's syndrome. The action of sodium hyaluronate is due not only to its protective role of a mechanical type exerted on the epithelial cells as a result of its viscoelasticity, in situations of reduced tear production, but also to the positive effects of its particular biological function on corneal epithelial cells by stimulating their migration *(*Exp. Eye Res., 1991;53:753-758*).*

Taurine (or 2-aminoethanesulphonic acid) is considered an amino acid, even though it does not possess the characteristic carboxyl group (COOH) but the SO₃H group. Taurine is only present in the animal realm, whereas vegetable foods do not possess this amino acid.

Vitamin E is the main antioxidant of the cell membranes, and is found in the human body in 4 forms consisting of α-tocopherol, β-tocopherol, γ-tocopherol and δ-tocopherol. Of these the α form is the most frequent in the retina and in plasma and is the one with the greatest antioxidant and free radical scavenging activity.

Previous uses of carnitine in the ophthalmological field are already known.

US Patent 5,037,851 describes the use of acetyl L-carnitine for the treatment of cataracts.

US 5,145,871 and 5,432,199 describe the use of acetyl D-carnitine for the treatment of glaucoma.

US 5,883,127 describes the use of acetyl L-carnitine for the treatment of maculopathy and macular degeneration.

Further uses of carnitine are also known.

In Res 1992;18(8):355-365 the use of L-carnitine in the cardiological field is described.

US 5,543,556 describes the use of acyl L-carnitine esters with gamma-hydroxybutyric acid for the inhibition of neuronal degeneration and in the treatment of coma.

US 5811457 describes the use of propionyl L-carnitine for the treatment of chronic obliterating arteriopathy.

None of the above-cited patents or publications describes or suggests the use of L-carnitine or of alkanoyl L-carnitine in combination with eledoisin for the treatment of diseases of the cornea.

In the medical field there is still a strongly perceived need for the availability of therapeutic agents or physiological supplement useful for the treatment of the above-mentioned corneal diseases.

### DETAILED DESCRIPTION OF THE INVENTION

It has now been found that L-carnitine and/or one or more alkanoyl L-carnitines selected from the group consisting of acetyl, propionyl, valeryl, isovaleryl, butiryl and isobutiryl L-carnitine, or one of their pharmaceutically acceptable salts, in combination with eledoisin, are useful agents for the preparation of an hypo-osmolal physiological supplement or medicament, in the form of eye-drops, in liquid or gel form, for the treatment of diseases of the cornea.

What is meant by pharmaceutically acceptable salt of L-carnitine is any salt of the latter with an acid that does not give rise to toxic or side effects.

These acids are well known to pharmacologists and to experts in pharmacy. Non-limiting examples of such salts are: chloride, bromide, orotate, aspartate, acid aspartate, acid citrate, magnesium citrate, phosphate, acid phosphate, fumarate and acid fumarate, magnesium fumarate, lactate, maleate and acid maleate, oxalate, acid oxalate, pamoate, acid pamoate, sulphate, acid sulphate, glucose phosphate, tartrate and acid tartrate, glycerophosphate, mucate, magnesium tartrate, 2-amino-ethanesulphonate, magnesium 2-amino- ethanesulphonate, methanesulphonate, choline tartrate, trichloroacetate, and trifluoroacetate.

What is meant by pharmaceutically acceptable salt of L-carnitine is also a salt approved by the FDA and listed in the publication Int. J. of Pharm. 33 (1986), 201-217.

One object of the present invention is the use of L-carnitine and/or of one or more alkanoyl L-carnitines, or a salt thereof, in combination with eledoisin, for the preparation of an ophthalmic physiological supplement or a medicament in the form of eye-drops, having an osmolality in a range of about 50 to about 250 mOsmols/kg, for the treatment of corneal diseases. Some preferred aspects of the invention include eye-drops having an osmolality in a range of about 100 to about 200 mOsmols/kg. More preferably, the eye drops have an osmolality of about 158 mOsmols/kg.

Examples of the corneal diseases include for example and without limitation, de-epithelializing diseases, dry eye syndrome; infective keratitis; acid or alkaline caustic damages; corneal abrasions and/or injuries due to mechanical action or contact lenses; degenerative disease of the corneal stroma such as acute or chronic keratoconus, stromal damages caused by refractive laser surgery; and dystrophic diseases, impairment of the transparency of the surface of the cornea caused by various types of infective keratitis (viral, bacterial and fungal), or by injuries that damage the structure of the various components constituting the cornea, such as, for instance, injuries of a mechanical, post-surgical and post-laser-refractive surgery type (such as, for example, haze); hereditary or degenerative diseases such as chronic and acute keratoconus.

A further object of the present invention is a physiological supplement or medicament for ophthalmic use, in the form of eye-drops, comprising as the active ingredient L-carnitine and or one or more alkanoyl L-carnitine, or a salt thereof, in combination with eledoisin and optionally ophthalmologically acceptable excipients and/or diluents;
in which:
- L-carnitine or the alkanoyl L-carnitine, or a salt thereof, is present preferably at a dose of about 0.2 to about 2.5% by weight, and most preferably at a dose of about 1% (amount as inner salt of L-carnitine);
- eledoisin is present preferably at a dose of about 0.004 to about 0.4% by weight, and most preferably at a dose of about 0.04%;

A further object of the present invention is a physiological supplement or medicament for ophthalmic use, in the form of eye-drops, comprising as the active ingredient L-carnitine, or one of its pharmaceutically acceptable salts in combination with eledoisin, humidifying agents such as sodium hyaluronate; antioxidants such as vitamin E; taurine, and optionally ophthalmologically acceptable excipients and/or diluents;
in which:
- L-carnitine is present preferably at a dose of about 0.2 to about 2.5% by weight, preferably is about 1%;
- eledoisin is present preferably at a dose of about 0.004 to about 0.4% by weight, preferably is about 0.04%;
- taurine is present preferably at a dose of about 0.1 to about 4% by weight, preferably is about 0.5%;
- sodium hyaluronate is present preferably at a dose of about 0.05 to about 1.5% by weight, preferably is about 0.2%;
- vitamin E is present preferably at a dose of about 0.05 to about 1.0% by weight, preferably is about 0.2%;
- the osmolality is preferably in a range of about 100 to about 250 mOsmols/kg, preferably is about 158 mOsmols/kg;
- the viscosity for eye drops in liquid form is preferably in a range of about 1 to about 50 mPa.sec.; preferably is about 17.8 mPa.sec.
- the viscosity for eye drops in gel form is preferably in a range of about 100 to about 5000 mPa.sec.; more preferably is about of 500 to about 2000 mPa.sec.; preferably is about 1000 mPa.sec.
- the pH is preferably in a range of about 5 to about 7.5, preferably is about 7.1.

A further object of the present invention is a kit comprising the active ingredients of the eye drops of the invention in the form of a powder and, separately, in the same or in a different container/vial, water or a liquid solution for ophthalmic use, in which the active ingredients (in the form of powder) and the water or the liquid solution can be easily put into contact and mixed together thus obtaining an ophthalmic composition for single use, avoiding the use of preservatives, such as BAK, and growth of microorganisms into the eye drops.

The expert in containers for ophthalmic use can easily suggest suitable containers/vials, which contain a powder and separately (in the same container) a liquid; or 2 different powders and a liquid; for single or multiple applications. Different containers/vials are also included in the present invention.

For purposes of the present invention, it will be understood by those of ordinary skill that the methods of treatment and use described herein are meant to include methods of treating human or animal eyes. Such methods include administering a medicament, for example, eye drops in accordance with the present invention, to a human or animal eye to provide medicinal benefit to the treated eye. The amount of the eye drops administered to the patient is generally described as an amount which is effect to treat, even temporarily and or symptomatically one or more of the conditions described herein. Thus the methods include administering 1 or more drops in the affected eye one or more times daily. The clinician of ordinary skill will, of course, be able to determine optimum dosing based on assessment of the clinical condition and strength of the ingredients included in the medicament.

Reference is made herein to medicaments in the form of eye drops. It should be understood that for purposes of the present invention that eye drops include solutions, suspensions, gels, creams and ointments intended for ophthalmic use.

The eye-drops according to the present invention may additionally contain further antioxidants, vitamins, minerals, Borage oil; epithelializing and anti-angiogenic agents; humidifying agents; inorganic elements; regulators of the cellular osmolality; antibiotics; anti-inflammatory agents, antiviral and antifungal agents. Such other components which may be included in the medicaments of the present invention may further include, without limitation, buffering agents, tonicity adjusting agents, ophthalmically acceptable preservatives, pH adjusting agents, components commonly found in artificial tears, such as one or more electrolytes, and the like and mixtures thereof. It will be further understood that all ingredients included in the medicaments of the present invention are preferably ophthalmically acceptable and can be chosen from materials which are conventionally employed in ophthalmic compositions, for example, compositions used to treat eyes afflicted with dry eye syndrome, artificial tear formulations and the like.

The term "ophthalmologically acceptable" with respect to a formulation, medicament, composition or ingredient herein means having no persistent detrimental effect on the treated eye or the functioning thereof, or on the general health of the subject being treated. It will be recognized that transient effects such as minor irritation or a "stinging" sensation are common with topical ophthalmic administration of drugs and the existence of such transient effects is not inconsistent with the formulation, composition or ingredient in question being "ophthalmically acceptable" as herein defined. However, preferred formulations, medicaments, compositions and ingredients are those that cause no substantial detrimental effect, even of a transient nature.

Aside from the amounts for each of the ingredients described herein, it will be understood that the compositions will include amounts generally understood in the art as being effective concentrations for such ingredients and as readily apparent to those of ordinary skill.

The following examples illustrate the invention.

### EXAMPLE 1

A double blind clinical trial in which 40 patients (8 males and 32 females aged from 56 to 80 years; mean age: 67 ± 10 years).

Said patients were suffering from primary open-angle glaucoma (POAG) treated with eye drops (for treating glaucoma) containing benzalkonium chloride (BAK).

Such glaucoma treatment (due to the presence of BAK in the eye drops administered), such patients were also suffering from dry eye disease.

It is well known to the skilled in the art that ophthalmic preservatives have many unwanted adverse effects, the severity of which is associated with the duration of medication, the types and concentrations of preservatives used in the medications and the number of eye drops per day administered to the patient.

### Inclusion criteria:

Patients with POAG diagnosis;
older than 40 years;
normal automated perimetry;
0.5 Cup/Disc;
compensated ocular tension; and
use of eye drops containing BAK for at least 6 months.

### Exclusion criteria:

Patients with systemic (i.e. connective, cardio-circulatory, defective metabolism disease) or ocular diseases which could modify the ocular surface (i.e. corneal dystrophy, rosacea, meibomian gland dysfunction), absolute central automated perimetry defects or subjects who underwent eye surgery within 6 months.

Patients were then divided into 2 groups.

**Group 1:** patients were treated 3 times a day for 15 days with the eye drops of the invention having the following composition:
- L-carnitine 1.017%;
- eledoisin 0.04%;
- taurine 0.49%;
- sodium hyaluronate 0.2% (0.2 g/100 mL);
- vitamin E acetate 0.2% (0.2 g/100 mL);
- EDTA 0.05 g/ 100 mL;
- Cromophor RH 40 2g/100 mL;
having a viscosity 17.8 mPa·sec, pH 7.1 and osmolality 158 mOsm/kg.

**Group 2:** patients were treated 3 times a day for 15 days with a saline solution comprising:
- EDTA 0.100 g/ 100 mL;
- N-hydroxymethyl glicynate 0.002 g/100 mL,
having a viscosity 2.0 mPa·sec, pH 7.2 and osmolality 300 mOsm/kg.

All the patients filled out a 12 questions form, Ocular Surface Disease Index (OSDI), concerning the severity of the symptoms and their impact on daily activities with total score from 0 to 48 and OSDI index from 0 to 100. In a second time, the subjects underwent the following tests: Fluorescein Breakup Time (FBUT), Ocular Protection Index (OPI), and Schirmer Test 1 (ST).

Tests were executed at time 0 (T0) and then repeated at the end of the therapy (T1).

OPI test assesses the risk of damage of the ocular surface and it is expressed as the ratio between FBUT, that measures the instability of the tear film, and blink interval (BI) calculated by dividing 60 for the number of eye blinks that the patient performs in 1 minute, while reading the Early Treatment Diabetic Retinopathy Study (ETDRS) charts. If the score is < 1 the patient can develop corneal damage. On the other hand, if the score is ≥ 1 there is no risk to develop corneal damage.

The FBUT test assesses the instability of the tear film. After a complete eye blink the tear film becomes gradually thinner until it breaks up creating random dry areas on the corneal epithelium. The score in terms of seconds represents the FBUT. After the instillation of fluorescein, the time between the last eye blink and the appearance of the first dry area identifies the break-up time and expresses the stability of the tear film. The use of a yellow filter (Kodak Wratten 12) improves the visibility of the breaking point in the fluorescent tear film. The cut-off for the FBUT to diagnose dry eye disease is less than 10 seconds, but more recently values between 5 and less than 10 seconds have been adopted, when small amounts of fluorescein are instilled (5 microliter of a 2% fluorescein solution).

The Schirmer Test 1 (ST) helps for the evaluation of the total tear secretion (basal and reflected). It is obtained by putting a strip of absorbent graduated paper in the conjunctival sac on the lower eyelid as described in Pescosolido N.: Le alterazioni del film lacrimale. In Stendler P.: "il sistema lacrimale", Fabiano editore, Canelli (AT), 2000; pag. 237-330. The patient should keep his eyes open and after 5 minutes it is possible to read the result. The length of the portion of the strip wet by tears provides a measure of tear secretion. Values for over 15mm/5' are considered as normal, values less than or equal to 15mm/5' suggest a tear hyposecretion.

### Results

At the end of therapy it was possible to evaluate the possible beneficial effects of eye drops of the invention.

In fact, after 15 days of treatment, patients of Group 1 showed a decrease of approximately 50% concerning the severity of symptoms (OSDI: T0=43; T1=25) and a significant improvement of the tests ST (T0=4.3 - T1=7.7), FBUT (T0=1.3 - T1=2.3) and OPI (T0=0.3 - T1=0.6).

On the other hand, in patients of Group 2 treated with placebo no significant improvement was found (OSDI: T0=35.6 - T1=36; ST: T0=4.7 - T1=4.5; FBUT: T0=4.7 - T1=4.6; OPI: T0=1.7 - T1=1.6).

The scores of the tests obtained in both groups are reported in terms of mean values in the following Table 1.

**TABLE 1**

| | | **GROUP 1** | | **GROUP 2** | |
|---|---|---|---|---|---|
| | | **Time (0)** | **Time (15 days)** | **Time (0)** | **Time (15 days)** |
| **OSDI** | Mean±SD | 43±17 | 25±7 | 35.6±10.1 | 36±12.1 |
| | Range | 20-70 | 15-38 | 18-65 | 15-65 |
| **ST** | Mean±SD | 4.3±1.7 | 7.7±2 | 4.7±2.1 | 4. 5±2.2 |
| | Range | 2-7 | 5-12 | 1-9 | 1-10 |
| **FBUT** | Mean±SD | 1.3±0.6 | 2.3±0.6 | 4.7±2 | 4.6±2.2 |
| | Range | 1-3 | 1-3 | 2-9 | 1-9 |
| **OPI** | Mean±SD | 0.3±0.1 | 0.6±0.2 | 1.7±1.1 | 1.6±1.1 |
| | Range | 0.2-0.7 | 0.3-1 | 0.3-4.4 | 0.3-4.4 |

### Discussion and conclusion

The results above reported show that patients with dry eye disease due to administration of eye drops with BAK for POAG, treated with the eye drops of the present invention had an important increase of ST, FBUT and OPI test scores and tear film stability. On the other hand the treatment according to the invention decreases the risk of corneal damage and the symptoms caused by dry eye disease only after 15 days of treatment. The results show how the eye drops of the invention improve the quality and the stability of the tear film by stimulating the secretion of the lacrimal gland.

L-carnitine and its alkanoyl derivatives are known compounds, the preparation process for which is described in US 4,254,053.

The physiological supplement or medicament according to the present invention may be bought with or without medical prescription.

The physiological supplement or medicament according to the present invention are composed of active ingredients which are familiar to operators in the medical field and already in use in clinical practice, and their pharmacotoxicological profiles are known.

Their procurement therefore is very easy, inasmuch as these are products which have been on the market now for a long time and are of a grade suitable for human or animal administration.

In the following are reported non limiting examples of compositions according to the present invention.

### Eye-drops

- L-carnitine 1.017%;
- eledoisin 0.04%;
- taurine 0.49%;
- sodium hyaluronate 0.2% (0.2 g/100 mL);
- vitamin E acetate 0.2% (0.2 g/100 mL);
- EDTA 0.05 g/ 100 mL;
- Cromophor RH 40 2g/100 mL;
- Viscosity 17.8 mPa·sec;
- pH 7.1;
- osmolality 158 mOsm/kg.

## Claims

1. Physiological supplement or medicament for ophthalmic use, in the form of eye-drops or gel, comprising L-carnitine and/or one or more alkanoyl L-carnitine, or a salt thereof, in combination with eledoisin.

2. The physiological supplement or medicament of claim 1, further comprising:
- taurine;
- sodium hyaluronate; and
- vitamin E.

3. The physiological supplement of claims 1 or 2, comprising:
- L-carnitine and/or one or more alkanoyl L-carnitine, or a salt thereof, in a dose of about 0.2 to about 2.5% by weight, preferably is about 1%;
- eledoisin in a dose of about 0.004 to about 0.4% by weight, preferably is about 0.04%;
- taurine in a dose of about 0.1 to about 4% by weight, preferably is about 0.5%;
- sodium hyaluronate in a dose of about 0.05 to about 1.5% by weight, preferably is about 0.2%;
- vitamin E in a dose of about 0.05 to about 1.0% by weight, preferably is about 0.2%.

4. Physiological supplement or medicament for ophthalmic use in the form of eye-drops or gel, comprising L-carnitine and/or one or more alkanoyl L-carnitine, or a salt thereof, in combination with eledoisin, for use in the treatment of corneal disease.

5. The physiological supplement or medicament of claim 4, further comprising:
- L-carnitine and/or one or more alkanoyl L-carnitine, or a salt thereof;
- eledoisin;
- taurine;
- sodium hyaluronate;
- vitamin E;
for use in the treatment of corneal disease.

6. The physiological supplement or medicament of claims 4 or 5 comprising:
- L-carnitine and/or one or more alkanoyl L-carnitine, or a salt thereof, in a dose of about 0.2 to about 2.5% by weight, preferably is about 1%;
- eledoisin in a dose of about 0.004 to about 0.4% by weight, preferably is about 0.04%;
- taurine in a dose of about 0.1 to about 4% by weight, preferably is about 0.5%;
- sodium hyaluronate in a dose of about 0.05 to about 1.5% by weight, preferably is about 0.2%;
- vitamin E in a dose of about 0.05 to about 1.0% by weight, preferably is about 0.2%;
for use in the treatment of corneal disease.

7. The physiological supplement or medicament of claims 1-6, optionally further comprising one or more ophtalmologically ecceptable excipients or diluents or mixture thereof.

8. The physiological supplement or medicament of claims 1-6 in which the salt of L-carnitine or alkanoyl L-carnitine is selected from the group consisting of chloride, bromide, orotate, aspartate, acid aspartate, acid citrate, magnesium citrate, phosphate, acid phosphate, fumarate and acid fumarate, magnesium fumarate, lactate, maleate and acid maleate, oxalate, acid oxalate, pamoate, acid pamoate, sulphate, acid sulphate, glucose phosphate, tartrate and acid tartrate, glycerophosphate, mucate, magnesium tartrate, 2-amino-ethanesulphonate, magnesium 2-amino-ethanesulphonate, methanesulphonate, choline tartrate, trichloroacetate, and trifluoroacetate.

9. The physiological supplement or medicament of claims 1-6 in which the alkanoyl L-carnitine is selected from the group consisting of acetyl, propionyl, valeryl, isovaleryl, butiryl or isobutiryl L-carnitine.

10. The physiological supplement or medicament of claims 1-6, optionally further comprising antioxidants; vitamin C; borage oil; epithelialising and anti-angiogenic agents; humidifying agents; inorganic elements; regulator of the cellular osmolarity; antibiotics; antiviral and antifungal agents.

11. The physiological supplement or medicament of claims 1-6, having an osmolality of from about 100 to about 250 mOsmol/kg, preferably is about 158 mOsmol/kg; and a pH of from about 5 to about 7.5, preferably pH is about 7.1.

12. The physiological supplement or medicament of claims 1-6 in liquid form, having a viscosity from about 1 to about 50 mPa·sec.; preferably is about 17.8 mPa·sec; or in a gel form, having a viscosity from about 100 to about 5,000 mPa·sec., preferably about 1,000. mPa·sec.

13. The physiological supplement or medicament of claims 1-6, in which the corneal disease is selected from the group comprising de-epithelialising diseases; dry eye syndrome; infective keratitis; acid or alkaline caustic damages; corneal abrasions and/or injuries selected from the group consisting of mechanical action or contact lenses; degenerative disease of the corneal stroma selected from the group consisting of chronic and acute keratoconus; stromal damages caused by refractive laser surgery; dystrophic diseases and impairment of the transparency of the surface of the cornea selected from the group consisting of infective keratitis, injuries and damages of the structure of the various components constituting the cornea such as aze; hereditary or degenerative diseases such as chronic and acute keratoconus.
